# EUROPÄISCHE PATENTSCHRIFT

(11) **EP 3 531 955 B1**
(45) Veröffentlichungstag und Bekanntmachung des Hinweises auf die Patenterteilung: **20.09.2023**
(21) Anmeldenummer: 17791332.4
(22) Anmeldetag: 20.10.2017
(51) Int. Cl.: A61B 46/10, A61B 46/00

(54) **STERILE ABDECKUNG FÜR EIN OPTISCHES GERÄT**
STERILE COVERING FOR AN OPTICAL DEVICE
HOUSSE STÉRILE POUR APPAREIL OPTIQUE

(30) Priorität: 28.10.2016 CH 14552016
(43) Veröffentlichungstag der Anmeldung: 04.09.2019
(73) Patentinhaber: Naviswiss AG, 5200 Brugg (CH)
(72) Erfinder: SCHWÄGLI, Tobias, 4500 Solothurn (CH); FINDEISEN, Charles, 5430 Wettingen (CH); HAURI, Thomas, 5722 Gränichen (CH)
(74) Vertreter: Herrmann, Johanna
(86) Internationale Anmeldenummer: PCT/EP2017/076805
(87) Internationale Veröffentlichungsnummer: WO 2018/077731

(56) Entgegenhaltungen:
- WO-A1-2017/079844
- US-A- 5 812 188
- US-A1- 2002 000 231
- US-B1- 8 146 825

## Beschreibung

Die Erfindung betrifft ein System, umfassend eine sterile Abdeckung sowie ein mit dieser sterilen Abdeckung abdeckbares optisches Gerät, beispielsweise ein handgehaltenes optisches Messsystem, welches insbesondere bei der computer - assistierten Chirurgie eingesetzt werden kann.

In der computer - assistierten Chirurgie eingesetzte handgehaltene, optische Messsysteme haben den Vorteil, dass sie für Messungen von beispielsweise Messmarkern freihändig vor das Messvolumen gehalten werden. Für Arbeitsschritte, für die keine Messungen notwendig sind, wird das Messsystem weggelegt. Für Arbeitsschritte, für die Messungen notwendig sind, wird das Messsystem wieder in die Hand genommen und vor das Messvolumen gehalten.

Die optischen Messsysteme müssen für deren Verwendung im unmittelbaren OP - Umfeld steril sein. Eine gängige Praxis dafür ist, dass optische Geräte oder Instrumente inklusive möglichen Kabeln in sterile, transparente Beutel (im Folgenden wird dafür die in der Fachwelt gebräuchliche englische Bezeichnung "Drape" verwendet) eingepackt werden, die eine zuverlässige Sperre zwischen dem nicht sterilen optischen Messsystem und dem sterilen OP - Umfeld sicherstellen. Manche optischen Messsysteme benötigen zusätzlich ein Fenster für die Beleuchtung des Messfeldes oder für bildgebende Systeme. Dafür wird ein optisches Fenster in eine Öffnung des Drapes eingefügt. Fenster und Drape sind so miteinander verbunden, dass eine zuverlässige Sperre zwischen dem nicht sterilen optischen Gerät oder Instrument und dem sterilen OP - Umfeld besteht.

Die Anforderungen an eine sterile Abdeckung umfassend ein Fensterelement sowie eine Hülle, das insbesondere für handgehaltene, optische Messsysteme geeignet ist, sind mannigfaltig. Im Folgenden wird dies am Beispiel eines handgehaltenen optischen Kamerasystems für die Messungen von Messmarkern diskutiert. So muss das Fensterelement stabil und auf definierte Weise am Kamerakörper befestigt werden können, so dass beispielsweise die vorgängig ermittelten Parameter für die Kamerakalibrierung gültig bleiben. Die Abdeckung muss mit gängigen Methoden wie Gammabestrahlung sterilisierbar sein. Bei Verwendung einer im Kamerasystem integrierten aktiven Beleuchtung kann ein nicht definiert oder stabil angebrachtes Fensterelement ein nicht erwünschtes direktes optisches Übersprechen von der Beleuchtung zum bildgebenden System begünstigen. Insbesondere ist wichtig, dass sich das Fensterelement auch beim Anstossen (absichtlichen oder unabsichtlich) des Kamerasystems an Gegenständen nicht aus der Befestigung löst. Zudem ist es von Vorteil, dass sich die sterile Abdeckung nach Gebrauch einfach und schnell wieder vom Kamerasystem entfernen lässt. Üblicherweise werden sterile Abdeckungen einmal verwendet (engl. "Single-Use"). Deshalb sind kostengünstig hergestellte sterile Abdeckungen anzustreben.

Der Stand der Technik für Drape - Systeme für handgehaltene optische Geräte wird beispielhaft in der Patentschrift US 8146825 B2 (Prpa) beschrieben. Eine mit federnden Zungen ausgestattete Fensterkappe wird auf das optische Gerät (in diesem Fall ist es ein Barcode - Leser) mit wenig Druck aufgesetzt. Das Entfernen des Fensters erfolgt umgekehrt durch einfaches Ziehen an der Kappe. Das Drape ist seitlich an der Fensterkappe angebracht. Die Bedienung des optischen Geräts, beispielsweise eines Handscanners, ist auch mit dem installierten Drape - System möglich. In der Patentschrift von Prpa wird jedoch nicht näher darauf eingegangen, wie das Fenster beim mechanischen Anstossen gesichert ist und ob die Messfähigkeit des optischen Geräts in jedem Fall sichergestellt wird.

Der Stand der Technik für Drape - Systeme für optische, nicht handgehaltene (das heisst stationäre) Systeme wird in zahlreichen Patentschriften und Patentanmeldungen beschrieben, wie beispielsweise in der bereits genannten US 8146825 B2 (Prpa), in US 2014/0261456 A1 (Stryker Corporation) oder in US 20100076306A1 (Daigneault). Auch in diesen Patentschriften wird nicht näher darauf eingegangen, wie das Fensterelement beim mechanischen Anstossen gesichert ist, um die Messfähigkeit des Messsystems sicherzustellen. In der Patentschrift US 9168103 B2 (Intellijoint Surgical Inc.) wird mittels einer zusätzlichen Klammer das Fensterelement am Kamerasystem befestigt.

Beim beschriebenen Stand der Technik wird die sichere Befestigung der sterilen Abdeckung am Messsystem hauptsächlich durch die sterile Abdeckung selber sichergestellt, wie beispielsweise durch im Fensterelement direkt integrierte Schnappmechanismen oder zusätzliche Hilfsmittel wie Klammern, Schraubverschlüsse oder elastische Bänder, was die Kosten für die Herstellung der sterilen Abdeckung Betriebskosten durch die teure Abdeckung beträchtlich. Wahlweise kann die Abdeckung gemäss US2002/0000231 A1 durch ein Gummiband oder ein elastisches Band gemäss US8146825 B1 auf einem Schutzzylinder mit Sichtfläche oder einem Schutzkonus mit Sichtfläche gesichert werden. Die Abdeckung kann gemäss US8146825 B1 auch mittels eines nicht näher definierten Schnappelements an der Seitenwand des Zylindermantels des Schutzzylinders oder Kegelmantels des Schutzkonus befestigt werden. Gemäss der US5812188 A wird ein Klebeband zum Verschliessen eines Drape-Beutels verwendet. Aufgabe der vorliegenden Erfindung ist es, ein System für ein optisches Gerät für die computer - assistierte Chirurgie weiterzuentwickeln, welches insbesondere gegen ein Verrutschen gesichert ist. Insbesondere soll die sterile Abdeckung des Systems einfach und schnell montierbar und austauschbar sein. Insbesondere soll die Herstellung der sterilen Abdeckung einfach und kostengünstig erfolgen können, sodass die Gesamtkosten des Systems für den Anwender reduziert werden können.

Die Aufgabe wird durch die Merkmale des Anspruchs 1 gelöst. Vorteilhafte Weiterbildungen ergeben sich aus den abhängigen Patentansprüchen.

Wenn der Begriff "beispielsweise" in der nachfolgenden Beschreibung verwendet wird, bezieht sich dieser Begriff auf Ausführungsbeispiele und/oder Ausführungsformen, was nicht notwendigerweise als eine bevorzugtere Anwendung der Lehre der Erfindung zu verstehen ist. In ähnlicher Weise sind die Begriffe "vorzugsweise", "bevorzugt" zu verstehen, indem sie sich auf ein Beispiel aus einer Menge von Ausführungsbeispielen und/oder Ausführungsformen beziehen, was nicht notwendigerweise als eine bevorzugte Anwendung der Lehre der Erfindung zu verstehen ist. Dementsprechend können sich die Begriffe "beispielsweise", "vorzugsweise" oder "bevorzugt" auf eine Mehrzahl von Ausführungsbeispielen und/oder Ausführungsformen beziehen.

Die nachfolgende detaillierte Beschreibung enthält verschiedene Ausführungsbeispiele für die erfindungsgemässe Abdeckung. Die Beschreibung einer bestimmten Abdeckung ist nur als beispielhaft anzusehen. In der Beschreibung und den Ansprüchen werden die Begriffe "enthalten", "umfassen", "aufweisen" als "enthalten, aber nicht beschränkt auf" interpretiert.

Ein System umfasst ein optisches Gerät und eine sterile Abdeckung für das optisches Gerät, umfassend ein Fensterelement und eine Hülle. Die Hülle ist am Fensterelement angebracht, wobei die Hülle eine Öffnung aufweist, die durch das Fensterelement verschlossen ist. Eine sterile Abdeckung kann eine sterilisierbare Abdeckung umfassen. Eine Abdeckung kann durch ein Sterilisierungsverfahren sterilisiert werden, sodass eine sterile Abdeckung erhalten werden kann.

Die Hülle weist einen Randbereich auf, wobei der Randbereich auf dem Fensterelement angebracht ist. Dieser Randbereich weist einen Rand auf, der die Öffnung begrenzt. Die Öffnung ist somit vom Randbereich umgeben. Hierdurch kann die Hülle einfach mit dem Fensterelement verbunden werden, da der Randbereich eine definierte Begrenzung, den Rand, ausbildet, die sich für eine automatisierte Montage eignet. Der Rand kann insbesondere in einem Stanzverfahren hergestellt werden. Erfindungsgemäß sind die Hülle und das Fensterelement durch eine Klebe- oder eine Schweissverbindung verbindbar. Insbesondere weist das Fensterelement eine sterile Seite auf, wobei die Hülle auf der sterilen Seite angebracht ist. Erfindungsgemäß ist der Randbereich zumindest teilweise nicht unmittelbar an die Kanten des Fensterelements angrenzend angeordnet. Hierdurch wird ermöglicht, dass die am optischen Gerät angebrachten Befestigungsadapter oder das Verriegelungselement unter der Hülle verschwinden können. Wahlweise können mehrere Verriegelungselemente vorgesehen sein. Ein Verriegelungselement und ein Befestigungsadapter sind vorgesehen. Wahlweise können eine Mehrzahl an Verriegelungselementen oder eine Mehrzahl von Befestigungsadaptern vorgesehen sein. Die Hülle kann insbesondere als eine flexible Hülle ausgebildet sein. Daher können die Dimensionen der Befestigungsadapter und/oder Verriegelungselemente beliebig gewählt werden, was ermöglicht, eine sterile Abdeckung für eine Vielzahl unterschiedlicher Befestigungsadapter oder Verriegelungselemente zu verwenden. Nach einem Ausführungsbeispiel enthält das Fensterelement eine erste Schicht und eine zweite Schicht. Durch die Verwendung einer zweiten Schicht kann die Formstabilität des Fensterelements erhöht werden. Zumindest eine der ersten oder zweiten Schichten kann eine Verstärkungsschicht ausbilden. Zumindest eine der ersten oder zweiten Schichten kann ein Loch aufweisen, sodass Messsignale nicht durch optische Effekte, wie unterschiedliche Brechungsindices oder Reflexionsphänomene beeinträchtigt werden. Nach einem Ausführungsbeispiel können die erste und zweite Schicht miteinander durch eine Klebeverbindung verbunden sein. Nach einem

Ausführungsbeispiel kann das Fensterelement auf mindestens einer Seite bedruckt sein. Insbesondere kann das Fensterelement ein oder mehrere aufgedruckte Gebiete enthalten. Ein derartiges aufgedrucktes Gebiet kann eine Orientierungshilfe für die Aufnahme des Fensterelements in einem Befestigungsadapter oder in einem Verriegelungselement sein. Insbesondere kann ein aufgedrucktes Gebiet auch Streulicht einer Lichtquelle absorbieren, um eine optische Messung nicht zu verfälschen.

Die Erfindung umfasst ein System umfassend ein optisches Gerät und eine sterile Abdeckung nach einem der vorhergehenden Ausführungsbeispiele. Insbesondere kann das Fensterelement über einem Messelement des optischen Geräts angeordnet sein. Das optische Gerät weist einen Befestigungsadapter oder ein Verriegelungselement für das Fensterelement auf. Wahlweise können mehrere Verriegelungselemente vorgesehen sein. Insbesondere können ein Verriegelungselement und ein Befestigungsadapter vorgesehen sein. Wahlweise können eine Mehrzahl an Verriegelungselementen oder eine Mehrzahl von Befestigungsadaptern vorgesehen sein. Das Verriegelungselement und der Befestigungsadapter können auf einander gegenüberliegenden Seitenkanten des Fensterelements angebracht sein. Das Verriegelungselement kann einen Schnappmechanismus und/oder einen Entriegelungsmechanismus enthalten.

Das System kann für stationäre oder portable optische Geräte verwendet werden. Insbesondere kann das System als ein stationäres oder ein portables System ausgebildet sein.

Die Energieversorgung oder ein Datentransfer für das System kann über ein Kabel erfolgen. Alternativ kann die Energieversorgung des Systems autonom erfolgen, beispielsweise über Akkus, Batterien. Wenn das System ein Kabel zur Energieversorgung enthält, weist die sterile Abdeckung eine Öffnung zur Durchführung des Kabels auf. Die Öffnung ist vorzugsweise derart verschliessbar, dass die Aussenseite der sterilen Abdeckung steril gehalten werden kann, auch wenn die Innenseite der sterilen Abdeckung ein nicht-steriles optisches Gerät umgibt. Die Innenseite der sterilen Abdeckung ist gegenüberliegend zum optischen Gerät angeordnet. Ein System mit autonomer Energieversorgung oder autonomer Abdeckung umgeben. Insbesondere können mit dem optischen Gerät Bilddaten erzeugt werden, die lokal verarbeitbar sein können.

Insbesondere kann mittels eines Systems gemäss einem der vorhergehenden Ausführungsbeispiele sichergestellt werden, dass sämtliche Bedienungsfunktionen des optischen Geräts am Einsatzort ausgeführt werden können, ohne dass eine Kabelverbindung zu einer Kontrolleinheit erforderlich ist. Insbesondere können systemexterne Funktionen, beispielsweise das Speichern oder Drucken von Daten, über eine kabellose Verbindung realisiert werden. Das optische Gerät kann insbesondere ein Anzeigegerät umfassen, welches zur Anzeige von Resultaten, beispielsweise in Form von Text, Bildern, Videos geeignet ist.

Ein Vorteil der sterilen Abdeckung sowie des Systems gemäss der Erfindung besteht darin, dass einerseits die Befestigungsadapter und das Verriegelungselement für das Fensterelement am optischen Messsystem direkt angebracht sind und andererseits die sterile Abdeckung im Wesentlichen aus einer Kombination aus einem Fensterelement und einer Hülle, insbesondere einem am Fensterelement angebrachten Drape, beispielsweise in der Form eines Beutels oder Schlauchs besteht. Die üblicherweise verwendeten Hüllen bestehen beispielsweise aus Polycarbonat oder Polyethylen.

Ein System nach einem der vorhergehenden Ausführungsbeispiele kann ein Fensterelement enthalten, welches eine Kante umfasst, die eine Fase oder Rundung enthält.

Nach einem Ausführungsbeispiel kann die Hülle das Verriegelungselement oder den Befestigungsadapter abdecken.

Die Zeichnungen zeigen Ausführungsbeispiele für die sterile Abdeckung sowie das System umfassend das in die sterile Abdeckung eingepackte optische Gerät. Es zeigen:
Fig. 1: eine Frontseite eines optischen Geräts nach einem ersten Ausführungsbeispiel,
Fig. 2: eine Frontseite eines optischen Geräts nach einem zweiten Ausführungsbeispiel,
Fig. 3: ein Detail der Befestigung eines Fensterelements eines Systems gemäss einer ersten Variante im Betriebszustand,
Fig. 4: ein Detail der Befestigung eines Fensterelements des Systems gemäss Fig. 3 bei der Montage oder Demontage,
Fig. 5: das System nach der ersten Variante nach einem der vorhergehenden Ausführungsbeispiele,
Fig. 6: einen Schnitt durch das System gemäss Fig. 5,
Fig. 7: die Rückseite des Systems nach dem zweiten Ausführungsbeispiel,
Fig. 8: eine Übersichtsskizze eines Systems gemäss einer zweiten Variante,
Fig. 9: ein bedrucktes Fensterelement.
Fig. 10: ein optisches Gerät nach einem dritten Ausführungsbeispiel,
Fig. 11: eine vergrösserte Ansicht des optischen Geräts gemäss Fig. 10,
Fig. 12: die Frontseite des optischen Geräts gemäss Fig. 10 mit dessen Fenster,
Fig. 13: ein Detail des Fensterelements sowie des optischen Geräts in einer Aufsicht,
Fig. 14: ein Detail eines Verriegelungselements,
Fig. 15: ein Detail des Fensterelements sowie des optischen Geräts in einer perspektivischen Darstellung,
Fig. 16: ein Detail eines Federelements für das Verriegelungselement gemäss Fig. 14,
Fig. 17: zeigt eine Seitenansicht des optischen Geräts, des Fensterelements sowie des Verriegelungselements gemäss Fig. 13,
Fig. 18: zeigt ein Detail der Fig. 17,
Fig. 19: einen Teilschnitt durch ein Ausführungsbeispiel eines Fensterelements,
Fig. 20: eine Seitenansicht des Fensterelements gemäss Fig. 19,
Fig. 21: eine Ansicht eines Ausführungsbeispiels eines Fensterelements.

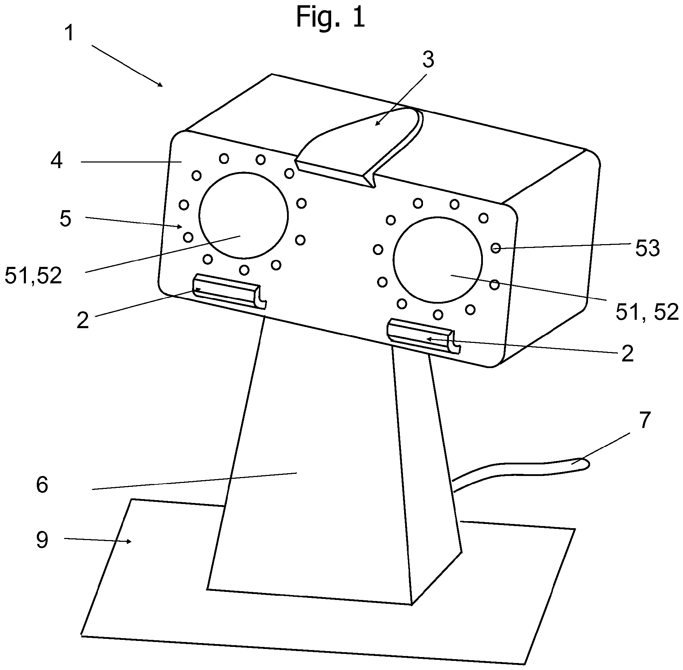

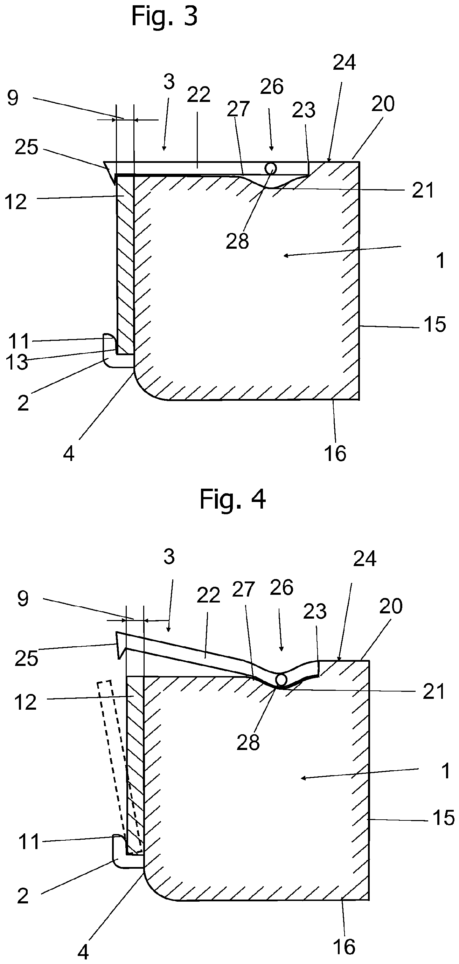

Fig. 1 zeigt eine Übersichtsskizze eines optischen Geräts 1 am Beispiel eines optischen Stereo - Kamerasystems enthaltend einen Befestigungsadapter 2 und ein Verriegelungselement 3 für ein Fensterelement 12 einer sterilen Abdeckung 10, wobei die sterile Abdeckung 10 und das Fensterelement 12 in der vorliegenden Darstellung weggelassen sind. Die sterile Abdeckung 10 und das Fensterelement 12 sind beispielsweise in einer der Fig. 3 bis Fig. 5 gezeigt. Die Optikseite oder Frontseite 4 des optischen Geräts 1 enthält nach dem vorliegenden Ausführungsbeispiel ein zum optischen Gerät 1 gehöriges Fenster 5. Das Fenster 5 kann aus zwei kreisförmigen Öffnungen für die beiden bildgebenden Systeme Objektiv 51 und Bildsensor 52 bestehen. Das Fenster 5 kann eine LED Ringanordnung 53 für die aktive Beleuchtung eines nicht dargestellten Messvolumens enthalten. Gemäss dem vorliegenden Ausführungsbeispiel sind zwei Befestigungsadapter 2 vorgesehen. Anstelle von zwei Befestigungsadaptern 2 kann auch ein einziger Befestigungsadapter oder es können mehr als zwei Befestigungsadapter vorgesehen sein, was zeichnerisch nicht dargestellt ist. Die Verwendung von zwei Befestigungsadaptern 2 hat den Vorteil, dass das Fensterelement 12 der sterilen Abdeckung 10 in seiner Lage eindeutig festgelegt ist. Das Verriegelungselement 3 dient der temporären Befestigung des Fensterelements 12 der sterilen Abdeckung 10 am optischen Gerät 1.

Die Skizze wird durch einen Standfuss 6 auf einem Tisch 9 und ein Kabel 7 für Energie und Datentransfer zum hier nicht gezeigten Energieversorgungs-, Auswerte- und Anzeigemodul beispielhaft vervollständigt. Alternativ kann das Kabel 7 entfallen. Die Energieversorgung kann im optischen Gerät 1 integriert sein. Das optische Gerät 1 kann mit externen Modulen drahtlos kommunizieren. Der Standfuss 6 kann auch als Adapter ausgebildet sein oder einen Adapter enthalten, um das optische Gerät 1 fest mit einer Auflage oder einem Objekt zu verbinden, was zeichnerisch nicht dargestellt ist. Die äusseren Oberflächen des optischen Geräts 1 sind sauber aber üblicherweise nicht steril.

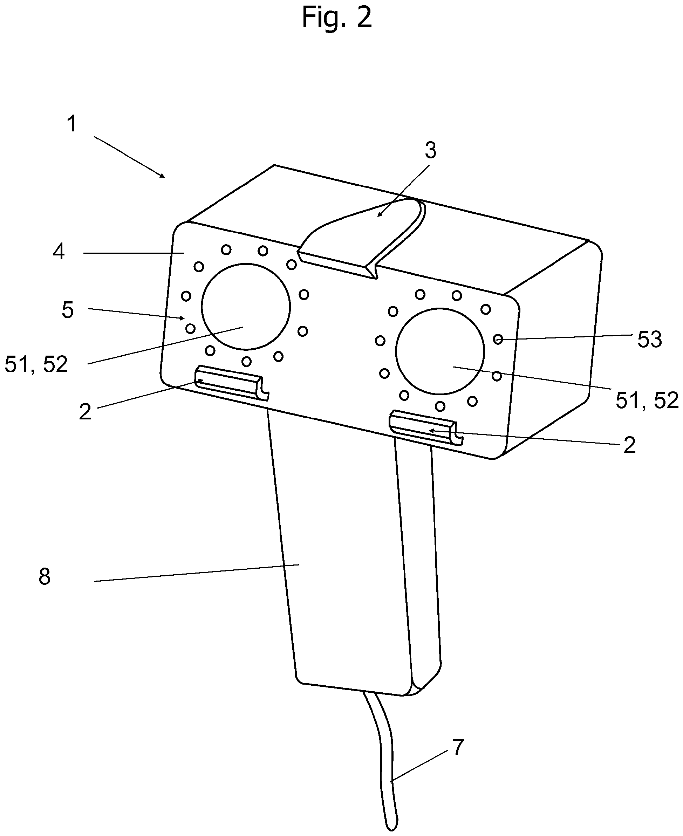

Fig. 2 zeigt eine Übersichtsskizze eines optischen Geräts 1 am Beispiel eines handgehaltenen optischen Stereo - Kamerasystems enthaltend einen Befestigungsadapter 2 und ein Verriegelungselement 3 für ein Fensterelement 12 einer sterilen Abdeckung 10. Die sterile Abdeckung 10 und das Fensterelement 12 sind in der Fig. 2 nicht dargestellt. Die Optikseite oder Frontseite 4 des optischen Geräts 1 enthält nach dem vorliegenden Ausführungsbeispiel ein zum optischen Gerät 1 gehöriges Fenster 5. Das Fenster 5 kann aus zwei kreisförmigen Öffnungen für die beiden bildgebenden Systeme Objektiv 51 und Bildsensor 52 bestehen. Das Fenster 5 kann eine LED Ringanordnung 53 für die aktive Beleuchtung eines nicht dargestellten Messvolumens enthalten. Die beiden Befestigungsadapter 2 und das Verriegelungselement 3 dienen der temporären Befestigung des Fensterelements. Die Skizze wird durch einen Handgriff 8 und ein Kabel 7 für Energie und Datentransfer zu einem hier nicht gezeigten Energieversorgungs- und Auswertemodul vervollständigt. Alternativ kann das Kabel entfallen. Nach dieser Variante kann die Energieversorgung im optischen Gerät 1 integriert sein. Das Auswertemodul kann vom optischen Gerät ermittelte Daten lokal analysieren. Das optische Gerät 1 kann mit externen Modulen drahtlos kommunizieren. Die äusseren Oberflächen des optischen Geräts 1 sind sauber aber üblicherweise nicht steril. In der computer - assistierten Chirurgie eingesetzte handgehaltene optische Geräte 1 haben den Vorteil, dass sie für Messungen von beispielsweise Messmarkern (hier nicht gezeigt) freihändig vor das Messvolumen gehalten werden. Für Arbeitsschritte, für welche keine Messungen notwendig sind, wird das optische Gerät 1 weggelegt. Für Arbeitsschritte, für die Messungen notwendig sind, wird das optische Gerät 1 wieder in die Hand genommen und vor das Messvolumen gehalten.

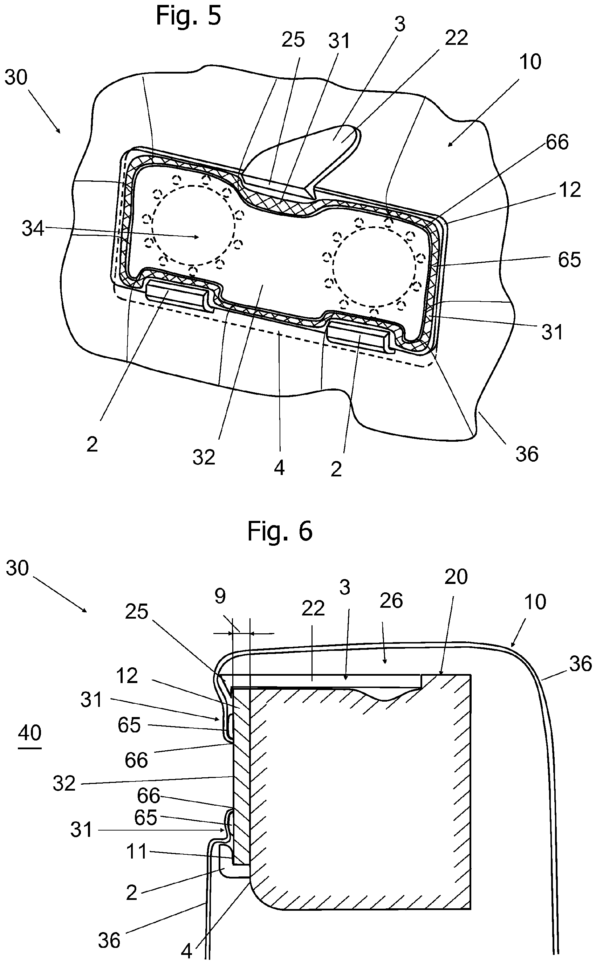

Fig. 3 zeigt die Befestigung eines Fensterelements 12 einer sterilen Abdeckung 10 eines Systems 30 gemäss Fig. 5 mittels eines Befestigungsadapters 2 sowie eines Verriegelungselements 3 an der Frontseite 4 des optischen Geräts 1 in einer Schnittdarstellung. Die Breite des Spalts zwischen der Frontseite 4 und der Innenseite des Befestigungsadapters 2 entspricht gerade der Dicke 9 des Fensterelements 12. Zumindest ein Teil 11 der Innenseite 13 des Befestigungsadapters 2 kann abgeschrägt sein, um das Einführen des Fensterelements 12 zu erleichtern. Unter dem Begriff «abgeschrägt» wird verstanden, dass die Innenseite 13 zumindest teilweise einen Neigungswinkel relativ zu der Ebene des Fensterelements 12 im eingebauten Zustand einschliessen kann. Der Neigungswinkel kann bis zu 15 Grad betragen, vorzugsweise beträgt der Neigungswinkel maximal 5 Grad. Gemäss einer Variante kann die Innenseite 13 eine Krümmung aufweisen. Der Neigungswinkel der Krümmung kann insbesondere von 0 Grad bis auf 90 Grad ansteigen. Die Innenseite 13 erstreckt sich vom Boden bis zum Ende des Befestigungsadapters. Der Boden nimmt die in der Zeichnung als Unterkante dargestellte Kante des Fensterelements 12 auf. Im Betriebszustand kann die Unterkante jede beliebige Raumposition einnehmen, je nach Positionierung des optischen Geräts 1.

Das optische Gerät 1 weist in der vorliegenden Darstellung eine Oberseite 20 auf. Die Oberseite 20 erstreckt sich zwischen der Frontseite 4 und der Rückseite 15. Gegenüberliegend zur Oberseite 20 befindet sich gemäss der Anordnung der Darstellung der Fig. 3 eine Unterseite 16. Die Oberseite 20 muss im Betriebszustand nicht notwendigerweise oben angeordnet sein, die Unterseite 16 muss im Betriebszustand nicht notwendigerweise unten liegen.

Die Oberseite 20 enthält das Verriegelungselement 3. Das Verriegelungselement 3 enthält ein Lamellenelement 22, welches insbesondere als eine federnde Lamelle ausgebildet sein kann. Nach einem nicht dargestellten Ausführungsbeispiel kann das Lamellenelement 22 zwischen einer Öffnungsposition und einer Schliessposition verschiebbar sein. Das Lamellenelement 22 enthält eine Basis 23, welche am optischen Gerät 1 befestigt ist. Insbesondere kann die Basis 23 am Gehäuse 24 des optischen Geräts 1 befestigt sein. Das der Basis 23 gegenüberliegende Ende 25 des Lamellenelements 22 kann als Hakenelement ausgebildet sein oder ein Hakenelement enthalten. Gemäss dem vorliegenden Ausführungsbeispiel überragt das Ende 25 des Lamellenelements 22 die Frontseite 4. Das Hakenelement weist einen Vorsprung auf, welcher in der Anordnung gemäss Fig. 3 die Oberkante des Fensterelements 12 umgreift.

Fig. 4 zeigt das Verriegelungselement 3 an der Oberseite 20 des optischen Geräts 1 im geöffneten Zustand. Das Lamellenelement 22 wird durch die Oberkante des anzubringenden Fensterelements 12 nach oben gedrückt und federt wieder in seine Ausgangsposition zurück, sobald das Fensterelement 12 an der Frontseite 4 anliegt. Das Hakenelement des Lamellenelements umgreift die Oberkante des Fensterelements 12, sodass das Fensterelement 12 durch den oder die Befestigungsadapter 2 und das Verriegelungselement 3 in Bezug auf das optische Gerät 1 in seiner Lage festgelegt, also temporär fixiert ist. Zum Entriegeln des Verriegelungselements 3 wird das Lamellenelement 22 an der Stelle 26 gegen die Oberseite 20 des optischen Geräts 1 bzw. dessen Gehäuse 24 gedrückt, so dass das Fensterelement 12 freigegeben wird. Das Ende 25 wird durch den an der Stelle 26 ausgeübten Anpressdruck angehoben, weil das Lamellenelement 22 eine Drehbewegung um eine Kante 27 ausführt. Der Teil des Lamellenelements 22 zwischen der Stelle 26 und dem Ende 25 wirkt somit als Hebel. Der Hebelarm, der sich zwischen dem Ende 25 und der Kante 27 erstreckt, ist länger als der Hebelarm, der sich zwischen der Kante 27 und dem tiefst gelegenen Punkt der Ausnehmung 21 erstreckt. Ein Teil des Lamellenelements 22, welcher sich vom Punkt 28, der oberhalb des tiefsten Punktes der Ausnehmung 21 gelegen ist, bis zur Basis 23 erstreckt, ist elastisch ausgebildet oder weist ein Gelenkelement auf. In dieser Konfiguration kann das Verriegelungselement 3 einen Entriegelungsmechanismus zur Freigabe des Fensterelements 12 enthalten. Der Benutzer kann das Fensterelement 12 abnehmen.

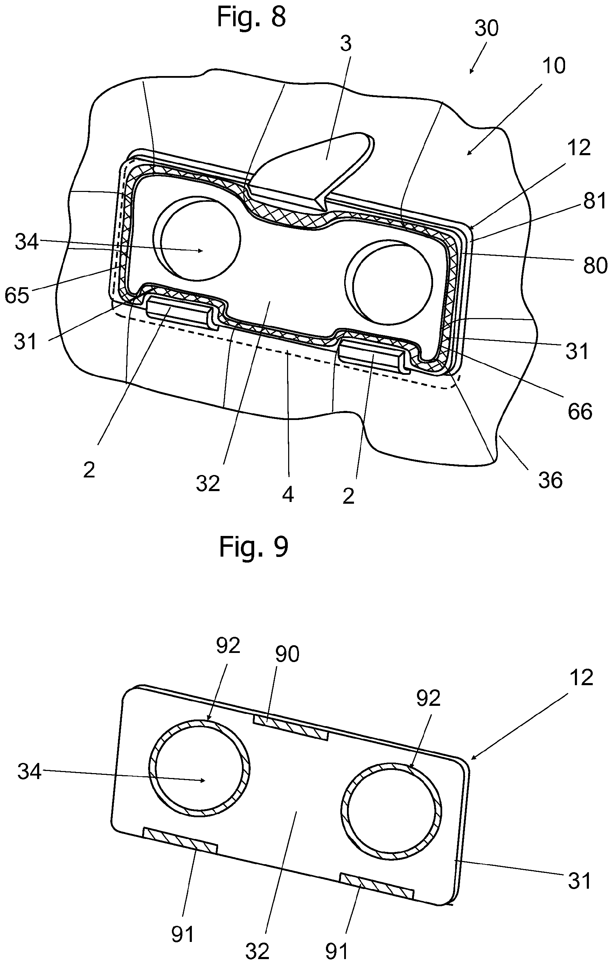

Fig. 5 zeigt ein System 30 umfassend eine sterile Abdeckung 10 umfassend ein Fensterelement 12 und eine Hülle 36, das sogenannte Drape, sowie ein optisches Gerät 1 gemäss Fig. 1. Das Fensterelement 12 der sterilen Abdeckung 10 ist mit dem optischen Gerät 1, von welchem nur die Frontseite 4 in strichlierten Linien dargestellt ist, mittels des Verriegelungselements 3 und den Befestigungsadaptern 2 verbunden. In diesem Beispiel wurde das Fensterelement 12 zur Befestigung in den Zwischenraum zwischen der Innenseite 13 des jeweiligen Befestigungsadapters 2 eingeführt, sodass die Unterkante des Fensterelements 12 von den Befestigungsadaptern 2 gehalten wird. Die Oberkante des Fensterelements 12 wird in Richtung der Frontseite 4 bewegt. Diese Bewegung ist im Wesentlichen eine Drehbewegung um eine entlang des Bodens des Befestigungsadapters 2 verlaufende Drehachse. Sobald die Oberkante des Fensterelements 12 das Ende 25 des Verriegelungselements 3 erreicht, wird durch den Anpressdruck auf das Ende 25 die Verriegelung gelöst. Das Ende 25 weist eine Vorderkante auf, welche einen Neigungswinkel in Bezug auf die Frontseite 4 aufweist. Die Vorderkante des Endes 25 ist dabei derart ausgebildet, dass sie durch Druck der Oberkante in der vorliegenden Darstellung von der Oberseite 20 weggedrückt wird. Sobald die Endposition des Fensterelements 12 erreicht ist, in welchem es auf der Frontseite 4 anliegt oder einen Minimalabstand zur Frontseite 4 aufweist, rastet das als Hakenelement ausgebildete Ende 25 des Lamellenelements 22 auf der Oberkante des Fensterelements 12 ein, wodurch ein Schnappmechanismus ausgebildet ist oder wird. Das Fensterelement 12 muss dabei ausreichend steif sein, so dass es sich beispielsweise nach dem Einklinken nicht wölbt. Hierzu kann das Fensterelement 12 gemäss Fig. 8 mindestens eine erste Schicht und eine zweite Schicht enthalten.

Am Fensterelement 12 ist die Hülle 36 angebracht. Als Materialien für das Fensterelement 12 können beispielsweise für das sterile Umfeld zugelassene Kunststoffe wie Polycarbonat oder Polyethylen von optischer Qualität eingesetzt werden. Die Hülle 36 weist eine Öffnung auf, wobei diese Öffnung von einem Randbereich 31 umgeben ist, der von einem Rand 66 begrenzt wird. Das heisst, dass an den Rand 66 der Öffnung der Randbereich anschliesst. Der Randbereich 31 weist eine Breite von 1 bis 10 mm auf, vorzugsweise 1 bis 5 mm. Diese Öffnung dient der Aufnahme des Fensterelements 12. Das heisst, die Öffnung wird durch das Fensterelement 12 verschlossen. Die Öffnung weist insbesondere eine kleinere Querschnittsfläche als das Fensterelement 12 auf. Das heisst, die Hülle 36 deckt das Fensterelement 12 in einem Randbereich 31 bis zum Rand 66 ab, wenn die Hülle 36 mit ihrer Öffnung und dem Randbereich 31 auf das Fensterelement 12 gelegt wird. Der Randbereich 31 der Öffnung wird mit der sterilen Seite 32 des Fensterelements 12 verbunden. Die sterile Seite 32 des Fensterelements 12 ist die dem sterilen Aussenbereich 40 zugewendete Seite des Fensterelements 12. Der Randbereich 31 und das Fensterelement 12 können durch eine Klebeverbindung 65 verbunden oder mittels Wärme oder Ultraschall verschweisst werden. Wenn eine Klebeverbindung 65 vorgesehen ist, enthält der Randbereich 31 Klebestellen. Wenn eine Schweissverbindung vorgesehen ist, enthält der Randbereich 31 eine Schweissnaht. Der Randbereich 31 kann bandförmig ausgebildet sein, wobei er insbesondere auf dem Fensterelement 12 derart angeordnet ist, dass er ausserhalb des in Fig. 1 oder Fig. 2 gezeigten Fensters 5 bzw. zumindest ausserhalb eines optisch durchlässigen Messbereichs des Fensters 5 verläuft. Der Randbereich 31 kann im Wesentlichen den Kanten des Fensterelements 12 folgen, ist aber im Bereich des oder der Befestigungsadapter 2 und des Verriegelungselements 3 zumindest teilweise in Richtung der Mitte des Fensters 5 bzw. des optisch durchlässigen Messbereichs des Messelements des optischen Geräts 1 verschoben. Der oder die Befestigungsadapter 2 und das Verriegelungselement 3 befinden sich ausserhalb des Randbereichs 31, wenn die Hülle 36 mit dem Fensterelement 12 verbunden ist. Die vom Rand 66 umschlossene Oberfläche 34 des Fensterelements 12 wird somit nicht von der Hülle 36 abgedeckt.

Das Zusammenfalten der Hülle 36 für den Gebrauch im sterilen OP Bereich ist der Fachwelt bekannt und nicht Gegenstand dieser Erfindung.

Fig. 6 zeigt einen Schnitt durch ein System 30 umfassend eine sterile Abdeckung 10 und ein optisches Gerät 1 nach dem in Fig. 3, Fig. 4 bzw. Fig. 5 gezeigten Ausführungsbeispiel. Der oder die Befestigungsadapter 2 und das Verriegelungselement 3 sind zusammen mit dem optischen Gerät 1 durch die sterile Abdeckung 10 vom sterilen Aussenbereich 40 getrennt. Auf der sterilen Seite 32 des Fensterelements 12 befindet sich der Randbereich 31. Im Randbereich 31 ist die Hülle 36 dicht mit dem Fensterelement 12 verbunden. Hierdurch wird sichergestellt, dass keine Stoffe vom optischen Gerät 1 in den sterilen Aussenbereich 40 gelangen können, Das Fensterelement 12 soll sich auch beim seitlichen Anstossen des optischen Geräts 1 an Gegenständen kaum aus der Befestigung lösen können, was beispielsweise durch Halterungen erfolgen kann, welche auch an den Seitenwänden des Fensterelements 12 angebracht sein können, aber in Fig. 6 nicht sichtbar sind.

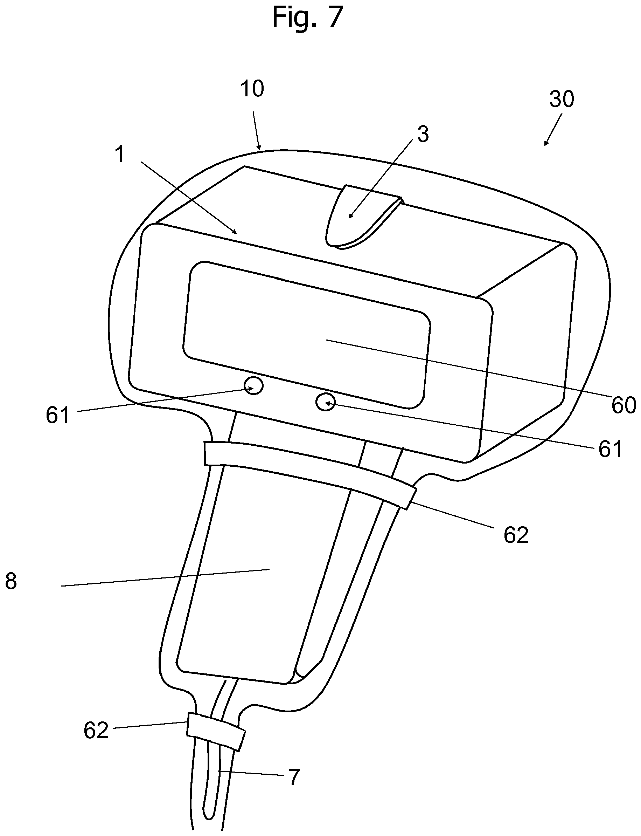

Fig. 7 zeigt eine Übersichtsskizze mit einer an einem optischen Gerät 1 angebrachten sterilen Abdeckung 10 mit Sicht auf ein Anzeigeelement 60 und Bedienelemente 61 des optischen Geräts 1. Die sterile Abdeckung 10 erlaubt die Benützung der Anzeige- und Bedienelemente. Ein Anzeigeelement 60 kann beispielsweise als Display ausgebildet sein. Ein Bedienelement 61 kann beispielsweise als Druckknopf ausgebildet sein. Ein Bedienelement kann im Display integriert sein, was zeichnerisch nicht dargestellt ist. Die sterile Abdeckung 10 wird an geeigneten Stellen mittels einem Verbindungsmittel 62 gehalten. Das Verbindungsmittel 62 kann ein Element aus der Gruppe der Gummibänder, Klebebänder, Tapes enthalten. Das optische Gerät kann somit mittels des oder der Verbindungsmittel 62 für den ergonomischen Gebrauch eingepackt werden.

Fig. 8 zeigt ein System 30 umfassend eine sterile Abdeckung 10, umfassend ein Fensterelement 12 und eine Hülle 36 gemäss einer Variante für ein optisches Gerät 1. Gleiche oder gleich wirkende Teile tragen dieselben Bezugszeichen wie in den vorhergehenden Ausführungsbeispielen und für die Beschreibung wird auf die vorhergehenden Ausführungsbeispiele verwiesen. Das Fensterelement 12 ist aus zwei Schichten 80, 81 aufgebaut, eine erste Schicht 80, welche die sterile Seite 32 des Fensterelements 12 enthält, und eine zweite Schicht 81, welche an die Frontseite 4 angrenzt. Insbesondere kann entweder die erste Schicht 80 oder die zweite Schicht 81 mindestens ein Loch aufweisen. Vorteilhafterweise enthält die erste Schicht 80 kein Loch, damit die sterile Seite 32 als zusammenhängende Schicht ausgebildet ist, sodass keine Undichtigkeiten auf der sterilen Seite 32 entstehen können. Die erste Schicht 80 ist somit als eine zusammenhängende Schicht ausgebildet. Die mit mindestens einem Loch ausgestattete Schicht 81 dient als mechanische Verstärkung oder Rahmen für das Fensterelement 12. Damit wird die benötigte Steifigkeit des Fensterelements 12 bei geringer Dicke erreicht. Die Dicke des Fensterelements 12, welches die erste und zweite Schicht 80, 81 umfasst, kann daher geringer sein als die Dicke eines Fensterelements 12, welches nur aus einer einzigen Schicht besteht.

Die Dicke des Fensterelements 12 kann nach jedem der Ausführungsbeispiele bis zu 5 mm betragen. Vorzugsweise beträgt die Dicke des Fensterelements maximal 3 mm. Besonders bevorzugt beträgt die Dicke des Fensterelements maximal 1.5 mm, da ein optisches Übersprechen für diesen besonders bevorzugten Bereich vermieden werden kann.

Fig. 9 zeigt eine Übersichtsskizze eines bedruckten Fensterelements 12. Die aufgedruckten Gebiete 90 und 91 sind Orientierungshilfen für das korrekte Aufsetzen des Fensterelements 12. Die beiden Ringe 92 reduzieren in diesem Beispiel das direkte optische Übersprechen von der aktiven Beleuchtung zum bildgebenden System des optischen Geräts 1, indem die Farbe der Ringe das Streulicht der aktiven Beleuchtung absorbiert. Beide Seiten des Fensterelements 12 können bedruckt sein. Zumindest der Aufdruck der Ringe 92 muss für den sterilen OP - Bereich geeignet sein.

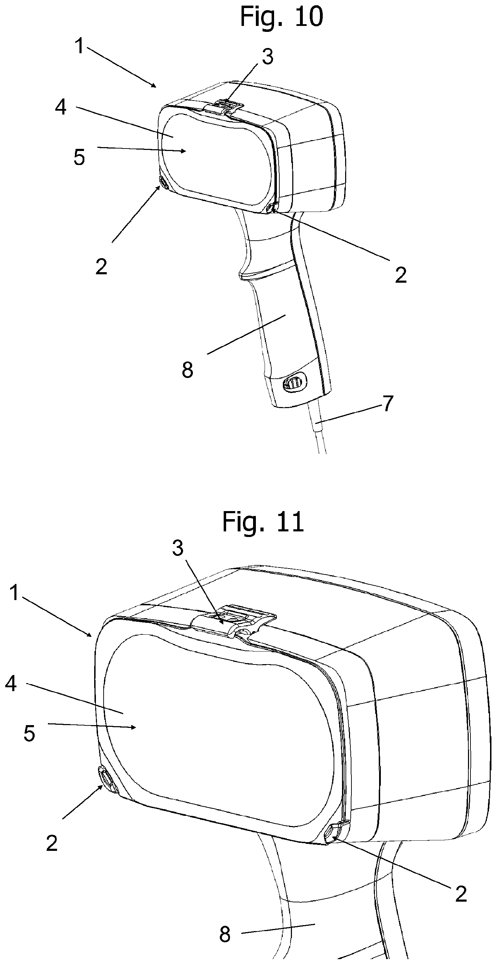

Fig. 10 zeigt eine Übersichtsskizze eines optischen Geräts 1 am Beispiel eines optischen Stereo - Kamerasystems gemäss einem dritten Ausführungsbeispiel enthaltend einen Befestigungsadapter 2 und ein Verriegelungselement 3 für ein Fensterelement 12 einer sterilen Abdeckung 10, wobei die sterile Abdeckung und das Fensterelement 12 in der vorliegenden Darstellung weggelassen sind. Die Optikseite oder Frontseite 4 des optischen Geräts 1 enthält nach dem vorliegenden Ausführungsbeispiel ein zum optischen Gerät 1 gehöriges Fenster 5, welches im Betriebszustand von einem Fensterelement 12 der sterilen Abdeckung 10 abgedeckt ist. Gemäss dem vorliegenden Ausführungsbeispiel sind zwei Befestigungsadapter 2 vorgesehen. Anstelle von zwei Befestigungsadaptern 2 kann auch ein einziger Befestigungsadapter vorgesehen sein, was zeichnerisch nicht dargestellt ist. Die Verwendung von zwei Befestigungsadaptern 2 hat den Vorteil, dass das Fensterelement 12 der sterilen Abdeckung 10 in seiner Lage eindeutig festgelegt ist. Das Verriegelungselement 3 dient der temporären Befestigung des Fensterelements 12 der sterilen Abdeckung 10 am optischen Gerät 1.

Die Skizze wird durch einen Handgriff 8 und ein Kabel 7 für Energie und Datentransfer zum hier nicht gezeigten Energieversorgungs-, Auswerte- und Anzeigemodul beispielhaft vervollständigt. Alternativ kann das Kabel 7 entfallen. Die Energieversorgung kann im optischen Gerät 1 integriert sein. Das optische Gerät 1 kann mit externen Modulen drahtlos kommunizieren. Der Handgriff 8 kann auch als Adapter ausgebildet sein oder einen Adapter enthalten, um das optische Gerät 1 fest mit einer Auflage oder einem Objekt zu verbinden, was zeichnerisch nicht dargestellt ist. Die äusseren Oberflächen des optischen Geräts 1 sind sauber, aber nicht steril.

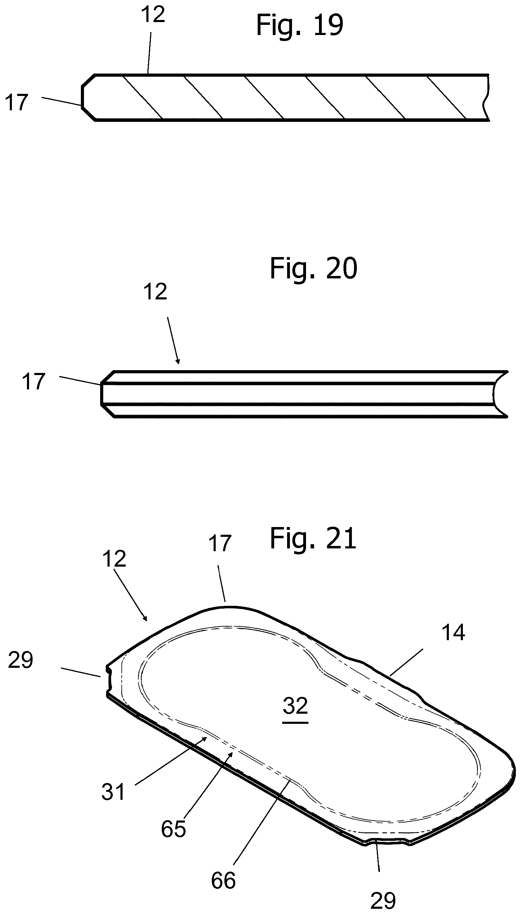

Fig. 11 zeigt das optische Gerät 1 der Fig. 10 im Detail. Insbesondere sind die Befestigungsadapter 2 sowie das Verriegelungselement 3 für das Fensterelement 12 besser sichtbar. Gemäss dem vorliegenden Ausführungsbeispiel erfolgt somit eine Befestigung des Fensterelements 12 an zwei Eckpunkten des Fensters 5 sowie einer Seitenkante des Fensters 5. Das Fensterelement 12 ist in Fig. 11 nicht dargestellt. Die Seitenkante des Fensterelements 12 wird durch das Verriegelungselement 3 gehalten. Die Seitenkante ist gemäss der vorliegenden Darstellung die obere Seitenkante. Sie liegt der unteren Seitenkante gegenüber, welche die Verbindungslinie zwischen Eckpunkten ausbildet, welche von den Befestigungsadaptern 2 gehalten wird. Das Fensterelement 12 kann eine abgeschrägte Ecke 29 enthalten, welche in Fig. 21 sichtbar ist. Die abgeschrägte Ecke 29 kann derart in den Befestigungsadaptern 2 aufgenommen sein, dass weder eine Verschiebung in Längsrichtung des Fensterelements 12 noch eine Verschiebung in Querrichtung des Fensterelements 12 ermöglicht ist. Daher ist die Ausrichtung des Fensterelements 12 in Bezug auf das Fenster 5 des optischen Geräts 1 vorgegeben, sodass im Betriebszustand das Fensterelement 12 genau über dem Fenster 5 des optischen Geräts 1 zu liegen kommen kann.

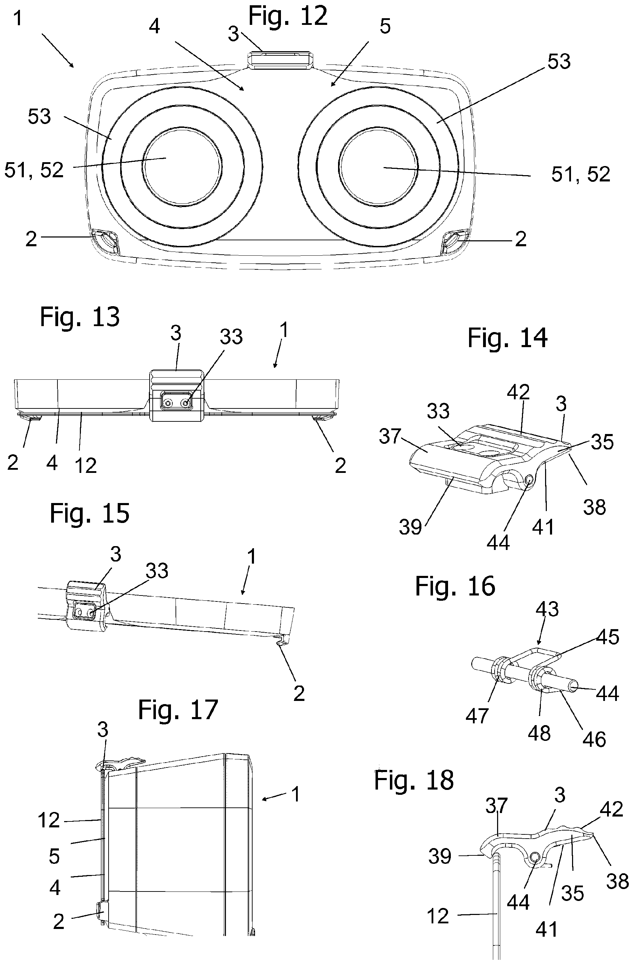

Fig. 12 zeigt die Frontseite 4 des optischen Geräts mit dessen Fenster 5. Das Fensterelement 12 ist in dieser Darstellung weggelassen. Die Optikseite oder Frontseite 4 des optischen Geräts 1 enthält somit nach dem vorliegenden Ausführungsbeispiel ein zum optischen Gerät 1 gehöriges Fenster 5. Das Fenster 5 kann aus zwei kreisförmigen Öffnungen für die beiden bildgebenden Systeme Objektiv 51 und Bildsensor 52 bestehen. Das Fenster 5 enthält mindestens einen Ring 53 für die aktive Beleuchtung eines nicht dargestellten Messvolumens. Der Ring 53 kann eine Lichtquelle enthalten.

Fig. 13 und Fig. 15 zeigen ein Detail des Fensterelements 12 sowie einen Teil des optischen Geräts 1 enthaltend das Verriegelungselement 3 in einer Aufsicht und einer perspektivischen Darstellung. Das Verriegelungselement 3 fixiert das Fensterelement 12 auf der Frontseite 4 des optischen Geräts 1. Das Verriegelungselement 3 ist mittels eines Befestigungselements 33 mit dem optischen Gerät verbunden. Das Befestigungselement 33 kann als eine Schraube, eine Stiftverbindung oder dergleichen ausgebildet sein, welche an einem Gehäuseelement des optischen Geräts 1 angebracht ist. Das Verriegelungselement 3 ist vorzugsweise derart mit dem optischen Gerät verbunden, dass es bezüglich des Befestigungselements 33 kippbar ausgebildet ist. Das Verriegelungselement 3 gemäss diesem Ausführungsbeispiel hat eine im Wesentlichen rechteckige Form in der Aufsicht.

Fig. 14 zeigt das Verriegelungselement 3 im Detail. Das Verriegelungselement 3 weist ein erstes Seitenelement 35 und ein zweites Seitenelement 37 auf. Das erste Seitenelement 35 erstreckt sich vom Befestigungselement 33 in Richtung des optischen Geräts 1. Das zweite Seitenelement 37 erstreckt sich vom Befestigungselement 33 in Richtung des Fensterelements 12. Das zweite Seitenelement 37 weist eine Kante 39 auf. Die Oberfläche des zweiten Seitenelements 37 kann zwischen dem Befestigungselement 33 und der Kante 39 eine Krümmung aufweisen. Die Krümmung ist gemäss diesem Ausführungsbeispiel derart ausgebildet, dass die Kante 39 den Rand des Fensterelements 12 umgreift, was in Fig. 15, Fig. 17 oder Fig. 18 sichtbar ist.

Das erste Seitenelement 35 weist eine Kante 38 auf. Die Kante 38 liegt nicht auf der Oberfläche des optischen Geräts 1 auf, sondern ist zu dieser Oberfläche in einem Abstand angeordnet, was in Fig. 15, Fig. 17 oder Fig. 18 sichtbar ist.

Zwischen der Kante 38 und dem Befestigungselement 33 erstreckt sich eine erste Seitenfläche 41, welche gegenüberliegend zur Oberfläche des optischen Geräts 1 angeordnet ist. Zwischen der Kante 38 und dem Befestigungselement 33 erstreckt sich eine zweite Seitenfläche 42, welche von der Oberfläche des optischen Geräts 1 weggerichtet ist. Zumindest eine der ersten oder zweiten Seitenflächen 41, 42 kann ebenfalls eine Krümmung aufweisen. Die zweite Seitenfläche 42 kann eine Profilierung aufweisen, beispielsweise Rillen oder Rippen. Die Profilierung kann dazu dienen, den Halt bei Betätigung des Verriegelungselements 3 zu verbessern.

Das Verriegelungselement 3 kann mittels eines Federelements 43 vorgespannt werden. Das Federelement 43 ist in Fig. 16 gezeigt. Das Federelement 43 umfasst ein erstes und ein zweites Bügelelement 45, 46 sowie eine erste und zweite Wicklung 47, 48, welche auf einer Achse 44 angeordnet sind. Die Achse 44 ist im Verriegelungselement 3 gehalten. Das erste Bügelelement 45 liegt auf der ersten Seitenfläche 41 des ersten Seitenelements 35 des Verriegelungselements 3 auf. Das zweite Bügelelement 45 liegt auf Oberfläche des optischen Geräts 1 auf. Das Federelement 43 ist derart vorgespannt, dass das Verriegelungselement 3 in der Verriegelungsposition verbleibt, solange das Verriegelungselement 3 nicht durch manuelle Betätigung entriegelt wird. Somit verbleibt das Fensterelement 12 sicher in der Schutzposition, in welcher das Fenster 5 des optischen Geräts 1 durch das Fensterelement 12 abgedeckt ist.

Das Federelement 43 hält das Verriegelungselement 3 gemäss Fig. 14 über zweite Seitenelement 37 sowie dessen Kante 39 geschlossen, das heisst das Federelement 43 ist Bestandteil eines Ausführungsbeispiels für einen Schnappmechanismus.

Fig. 17 zeigt eine Seitenansicht des optischen Geräts, des Fensterelements 12 sowie des Verriegelungselements 3 und des Befestigungsadapters 2.

Insbesondere zeigt Fig. 17 die Kante 39 des zweiten Seitenelements 37 des Verriegelungselements 3, welches den Rand des Fensterelements 12 umgreift und das Fensterelement 12 bezüglich der Frontseite 4 des optischen Geräts 1 derart fixiert, dass das Fenster 5 vom Fensterelement 12 abgedeckt wird. Das Fensterelement 12 kann insbesondere auf dem Fenster 5 aufliegen, sodass zwischen dem Fensterelement 12 und dem Fenster 5 kein Zwischenraum verbleibt.

Fig. 18 zeigt ein Detail der Fig. 17, in welchem das optische Gerät 1 weggelassen ist. Das Verriegelungselement 3 ist in Fig. 18 geschnitten dargestellt. Gemäss diesem Ausführungsbeispiel enthalten das erste Seitenelement 35 und das zweite Seitenelement 37 einen Hohlraum. Der Hohlraum kann zur Aufnahme eines Federelements ausgebildet sein, beispielsweise eines Federelements 43 gemäss Fig. 16.

Das erste Seitenelement 35 enthält die zweite Seitenfläche 42, welche ein Ausführungsbeispiel für einen Entriegelungsmechanismus ist. Der Entriegelungsmechanismus wird durch Druck auf die zweite Seitenfläche 42 aktiviert. Durch Druck auf die zweite Seitenfläche 42 in Richtung der Oberfläche des optischen Geräts 1 wird das Federelement 43 zusammengedrückt und das zweite Seitenelement 37 angehoben, sodass der Schnappmechanismus gelöst werden kann. Der Druck kann beispielsweise durch den Finger eines Benutzers des Systems ausgeübt werden. Die Federkraft des Federelements 43 ist vorzugsweise so eingestellt, dass die Federkraft durch Fingerdruck überwunden werden kann.

Ein Verriegelungselement 3 gemäss dem Ausführungsbeispiel gemäss einer der Fig. 13 bis 18 ist einfach zu bedienen. Wenn man das Fensterelement 12 vom optischen Gerät 1 entfernen möchte, kann man mit einem Finger einen Druck auf die zweite Seitenfläche 42 des Verriegelungselements 3 ausüben. Durch den manuellen Druck auf die zweite Seitenfläche 42 wird die Seitenfläche 42 um die Achse 44 gedreht, wobei das Federelement 43 von der ersten Seitenfläche 41 zusammengedrückt wird. Das erste Seitenelement 35 sowie dessen Kante 39 werden hierdurch von der Oberfläche des optischen Geräts 1 wegbewegt, in den vorliegenden Figuren 13 bis 18 angehoben. Das Fensterelement 12 kann in die Befestigungsadapter 2 eingeschoben werden und auf das Fenster 5 aufgelegt werden. Nach Platzierung des Fensterelements 12 auf dem Fenster 5 kann das Verriegelungselement 3 losgelassen werden, das heisst der manuelle Druck auf das Verriegelungselement 3 wegfallen. Das Federelement 43 wird entspannt, wodurch sich die zweite Seitenfläche 42 und die erste Seitenfläche 41 von der Oberfläche des optischen Geräts 1 wegbewegen und sich das zweite Seitenelement 37 in Richtung der oberen Seitenkante des Fensterelements 12 bewegt. Die Kante 39 des zweiten Seitenelements 37 überragt das Fensterelement 12. Das zweite Seitenelement 37 weist eine Krümmung auf, welche die obere Seitenkante des Fensterelements 12 teilweise umschliesst. Die Krümmung kann derart ausgeführt sein, dass die obere Seitenkante des Fensterelements 12 gegen das Fenster 5 bewegt wird, um auf dem Fenster 5 aufzuliegen. Durch die Form des zweiten Seitenelements 37 kann somit das Fensterelement 12 in Anschlag mit dem Fenster 5 gebracht werden, sodass das Fensterelement 12 unverrückbar auf dem Fenster 5 platziert werden kann. Das Verriegelungselement 3 kann wiederum durch Ausüben eines Drucks auf die zweite Seitenfläche 42 gelöst werden. Das zweite Seitenelement bewegt sich dann wieder von der oberen Seitenkante des Fensterelements 12 weg, wodurch das Fensterelement 12 vom Fenster 5 entfernt werden kann.

Alternativ besteht auch die Möglichkeit, das Fensterelement 12 durch Anwendung einer Zugkraft, die vom Fenster 5 weggerichtet ist, aus dem Verriegelungselement 3 zu lösen. Die obere Seitenkante des Fensterelements 12 übt einen Druck auf das zweite Seitenelement 37 aus, wodurch ein Zusammendrücken des Federelements 43 und ein Anheben des zweiten Seitenelements 37 erzwungen werden kann. Hierdurch kann das Fensterelement 12 besonders schnell vom Fenster 5 entfernt werden. Die Federkraft des Federelements 43 ist insbesondere so bemessen, dass das Fensterelement 12 einerseits ohne zu verrutschen auf dem Fenster gehalten werden kann und andererseits manuell entfernt werden kann, gegebenenfalls auch ohne eine Betätigung des Verriegelungselements 3. Die Form der Kante 39 und die Form der Kante 17 des Fensterelements 12 können derart aneinander angepasst sein, dass sie bei Anwendung einer Zugkraft aneinander vorbeigleiten können. Insbesondere kann die Kante 39 eine Krümmung aufweisen. Die Kante 17 kann eine Rundung oder eine Fase umfassen.

Fig. 19 zeigt einen Teilschnitt durch ein Ausführungsbeispiel eines Fensterelements 12. Die Kante des Fensterelements weist Fasen oder Rundungen auf, wodurch die Verletzungsgefahr bei Manipulation des Fensterelements 12 verringert ist. Zudem kann eine Hülle 36 (siehe z.B. Fig. 5) nicht durch eine Kante des Fensterelements 12 beschädigt werden, wenn diese Hülle versehentlich mit der Kante des Fensterelements 12 in Kontakt kommen sollte.

Fig. 20 zeigt eine Seitenansicht des Fensterelements 12 gemäss Fig. 19.

Fig. 21 zeigt eine Ansicht eines Ausführungsbeispiels eines Fensterelements 12. Das Fensterelement 12 weist vier Kanten auf. Zwei dieser Kanten begrenzen Ecken, die gemäss diesem Ausführungsbeispiel nicht als Spitze oder als gerundete Spitze ausgebildet sind, sondern schräg abgeschnitten, das heisst als abgeschrägte Ecke 29 ausgebildet. Die Form der abgeschrägten Ecken 29 ist besonders geeignet zur Aufnahme in einem entsprechenden Befestigungsadapter 2 gemäss dem in Fig. 10, 11 dargestellten Ausführungsbeispiel. Je nach Anzahl der Befestigungsadapter 2 kann eine entsprechende Anzahl an abgeschrägten Ecken 29 vorgesehen sein. Beispielsweise kann auch nur eine einzige abgeschrägte Ecke 29 vorgesehen sein, was zeichnerisch nicht dargestellt ist. Die Kante 17, welche den Umfang des Fensterelements 12 ausbildet und die vier Seitenkanten des Fensterelements 12 enthält, kann eine Fase oder eine Rundung enthalten. Bei Verwendung einer Kante 17, welche eine Rundung oder eine Fase enthält, kann die Verletzungsgefahr oder die Gefahr einer Schädigung des Systems, insbesondere der Hülle 36 oder des optischen Geräts 1 verringert werden. Zudem kann ein Gleiten der Kante 17 relativ zur Kante 39 wie oben beschrieben erfolgen, wenn auf das Fensterelement 12 eine Zugkraft ausgeübt wird, die vom Fenster 5 weggerichtet ist.

Eine der Seiten des Fensterelements 12 kann einen Vorsprung 14 enthalten. Der Vorsprung 14 kann dazu bestimmt sein, in einem Verriegelungselement 3 gemäss einem der vorhergehenden Ausführungsbeispiele aufgenommen zu werden. Der Vorsprung 14 kann gegenüberliegend der Seitenkante angeordnet sein, welche sich zwischen den beiden abgeschrägten Ecken 29 erstreckt. Das Fensterelement 12 kann eine Klebeverbindung 65 aufweisen. Die Klebeverbindung 65 befindet sich auf der sterilen Seite 32 des Fensterelements 12, das heisst auf der Seite des Fensterelements 12, die der sterilen Abdeckung 10 gegenüberliegt. Die Klebeverbindung 65 kann als doppelseitig klebende Folie ausgebildet sein. Die Klebeverbindung 65 dient der Befestigung der Hülle 36 auf der sterilen Seite 32 des Fensterelements 12. Anstatt einer Klebeverbindung 65 kann die Verbindung zwischen dem Fensterelement 12 und der Hülle 36 durch Ultraschallschweissen erzielt werden. Die Hülle 36 ist in Fig. 21 nicht dargestellt, sie kann gemäss einem der Ausführungsbeispiele gemäss einer der Fig. 5 bis 7 ausgebildet sein. Die Verbindung, die hier als Klebeverbindung 65 ausgebildet ist, aber nach anderen Ausführungsbeispielen als eine mittels Ultraschallschweissen hergestellte Verbindung ausgebildet sein kann weist eine Dichtigkeit gegenüber Flüssigkeiten, Gasen, Chemikalien, biologischen oder biogenen Materialien, insbesondere gegen Krankheitserreger, Mikroben, Viren, Bakterien, Prionen und dergleichen auf.

Der Vorsprung 14 kann achsensymmetrisch bezüglich einer Symmetrieachse des Fensterelements 12 angeordnet sein.

Das Fensterelement 12 nach einem der Ausführungsbeispiele kann eine Beschichtung enthalten, die insbesondere ein Element aus der Gruppe der antistatischen, Beschichtungen, schmutzabweisenden Beschichtungen, Antireflexionsbeschichtungen enthält. Insbesondere kann eine Antireflexionsbeschichtung vorgesehen sein, um Streulicht zu vermindern oder zu vermeiden.

Für den Fachmann ist offensichtlich, dass viele weitere Modifikationen zusätzlich zu den beschriebenen Ausführungsbeispielen möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist.

## Patentansprüche

1. System (30) umfassend ein optisches Gerät (1) und eine sterile Abdeckung (10) für das optische Gerät (1), wobei die sterile Abdeckung (10) ein Fensterelement (12) und eine Hülle (36) umfasst, wobei die Hülle (36) am Fensterelement (12) angebracht ist, wobei die Hülle (36) eine Öffnung aufweist, die durch das Fensterelement (12) verschlossen ist, wobei die Hülle (36) einen Randbereich (31) aufweist, wobei der Randbereich (31) auf dem Fensterelement (12) angebracht ist, wobei die Öffnung vom Randbereich umgeben ist, **dadurch gekennzeichnet, dass** die Öffnung eine kleinere Querschnittsfläche als das Fensterelement (12) aufweist, wobei das optische Gerät (1) einen Befestigungsadapter (2) oder ein Verriegelungselement (3) für das Fensterelement (12) aufweist, wobei die Hülle (36) und das Fensterelement (12) durch eine Klebeverbindung (65) oder eine Schweissverbindung verbindbar sind, wobei der Randbereich (31) zumindest teilweise nicht unmittelbar an die Kanten des Fensterelements (12) angrenzend angeordnet ist.

2. System nach Anspruch 1, wobei das Fensterelement (12) eine sterile Seite (32) aufweist, wobei die Hülle (36) auf der sterilen Seite (32) angebracht ist.

3. System nach einem der vorhergehenden Ansprüche, wobei das Fensterelement (12) eine erste Schicht (80) und eine zweite Schicht (81) enthält.

4. System nach Anspruch 3, wobei eine der ersten oder zweiten Schichten (80, 81) ein Loch aufweist.

5. System nach einem der Ansprüche 3 oder 4, wobei die erste und zweite Schicht (80, 81) miteinander durch eine Klebeverbindung verbunden sind.

6. System nach einem der vorhergehenden Ansprüche, wobei das Fensterelement (12) auf mindestens einer Seite bedruckt ist.

7. System (30) nach Anspruch 1, wobei das Fensterelement (12) über einem Messelement des optischen Geräts angeordnet ist.

8. System (30) nach Anspruch 1, wobei das Verriegelungselement (3) oder der Befestigungsadapter (2) auf einander gegenüberliegenden Seitenkanten des Fensterelements (12) angebracht sind.

9. System (30) nach einem der vorhergehenden Ansprüche 1 oder 8, wobei das Verriegelungselement (3) einen Schnappmechanismus oder einen Entriegelungsmechanismus enthält.

10. System (30) nach einem der vorhergehenden Ansprüche 1, 8 oder 9, wobei das Fensterelement (12) eine Kante (17) umfasst, die eine Fase oder Rundung enthält.

11. System (30) nach einem der vorhergehenden Ansprüche 1, 8 bis 10, wobei die Hülle (36) das Verriegelungselement (3) oder den Befestigungsadapter (2) abdecken kann.

## Claims

1. System (30) comprising an optical device (1) and a sterile covering (10) for the optical device (1) wherein the sterile covering (10) comprises a window element (12) and a shell (36) **characterized in that** the shell (36) is attached to the window element (12), wherein the shell (36) has an opening closed by the window element (12), wherein the shell (36) has an edge region (31), wherein the edge region (31) is mounted on the window element (12), wherein the opening being surrounded by the edge region, **characterized in that** the opening has a smaller cross-sectional area than the window element (12), wherein the optical device (1) comprises a mounting adapter (2) or a locking element (3) for the window element (12), wherein the shell (36) and the window element (12) are connectable by an adhesive (65) or a welded connection, wherein the edge region (31) is arranged at least partially not directly adjacently to the edges of the window element (12).

2. The system of claim 1, wherein the window element (12) is disposed with a sterile side (32), wherein the shell (36) is attached to the sterile side (32).

3. The system of one of the preceding claims, wherein the window element (12) comprises a first layer (80) and a second layer (81).

4. The system of claim 3, wherein one of the first or second layers (80, 81) comprises an opening.

5. The system of one of claims 3 or 4, wherein the first and second layer (80, 81) are joined together by an adhesive bond.

6. The system of one of the preceding claims, wherein the window element (12) is printed on at least one side.

7. The system (30) of claim 1, wherein the window element (12) is arranged above a measuring element of the optical device.

8. The system (30) of claim 1, wherein the locking element (3) or the fastening adapter (2) are mounted on opposite side edges of the window element (12).

9. The system (30) of one of claims 1 or 8, wherein the locking element (3) includes a snap mechanism or an unlocking mechanism.

10. The system (30) of one of claims 1, 8 or 9, wherein the window element (12) comprises an edge (17) containing a chamfer or a rounding.

11. The system (30) of one of claims 1 or 8 to 10, wherein the shell (36) can cover the locking element (3) or the fastening adapter (2).

## Revendications

1. Système (30) comprenant un dispositif optique (1) et une couverture stérile (10) pour le dispositif optique (1), dans lequel la couverture stérile (10) comprend un élément de fenêtre (12) et une coque (36) **caractérisée en ce que** la coque (36) est fixée à l'élément de fenêtre (12), dans laquelle la coque (36) a une ouverture fermée par l'élément de fenêtre (12), dans laquelle la coque (36) a une région de bord (31), dans laquelle la région de bord (31) est montée sur l'élément de fenêtre (12), dans laquelle l'ouverture est entourée par la région de bord, **caractérisé en ce que** l'ouverture a une section transversale plus petite que l'élément de fenêtre (12), dans lequel le dispositif optique (1) comprend un adaptateur de montage (2) ou un élément de verrouillage (3) pour l'élément de fenêtre (12), dans lequel la coque (36) et l'élément de fenêtre (12) peuvent être reliés par un adhésif (65) ou une connexion soudée, dans lequel la région de bord (31) est disposée au moins partiellement de manière non directement adjacente aux bords de l'élément de fenêtre (12).

2. Le système de la revendication 1, dans lequel l'élément de fenêtre (12) est disposé avec un côté stérile (32), dans lequel la coque (36) est attachée au côté stérile (32).

3. Le système de l'une des revendications précédentes, dans lequel l'élément de fenêtre (12) comprend une première couche (80) et une deuxième couche (81).

4. Le système de la revendication 3, dans lequel l'une de la première ou de la deuxième couche (80, 81) comprend une ouverture.

5. Le système de l'une des revendications 3 ou 4, dans lequel la première et la deuxième couche (80, 81) sont assemblées par une liaison adhésive.

6. Le système de l'une des revendications précédentes, dans lequel l'élément de fenêtre (12) est imprimé sur au moins une face.

7. Le système (30) de la revendication 1, dans lequel l'élément de fenêtre (12) est disposé au-dessus d'un élément de mesure du dispositif optique.

8. Le système (30) de la revendication 1, dans lequel l'élément de verrouillage (3) ou l'adaptateur de montage (2) sont montés sur les bords latéraux opposés de l'élément de fenêtre (12).

9. Le système (30) de l'une des revendications 1 ou 8, dans lequel l'élément de verrouillage (3) comprend un mécanisme d'encliquetage ou un mécanisme de déverrouillage.

10. Le système (30) de l'une des revendications 1, 8 ou 9, dans lequel l'élément de fenêtre (12) comprend un bord (17) contenant un chanfrein ou un arrondi.

11. Le système (30) de l'une des revendications 1 ou 8 à 10, dans lequel la coque (36) peut recouvrir l'élément de verrouillage (3) ou l'adaptateur de montage (2).
